# EUROPÄISCHE PATENTANMELDUNG

(11) **EP 4 283 625 A1**
(43) Veröffentlichungstag der Anmeldung: **29.11.2023**
(21) Anmeldenummer: 23175303.9
(22) Anmeldetag: 25.05.2023
(51) Int. Cl.: G16H 20/17, G16H 40/40, G16H 40/67

(54) **AUTHENTIFIZIERUNG VON PERSONEN ZUR EINSTELLUNG VON ZUMINDEST EINER INFUSIONSPUMPE**

(30) Priorität: 25.05.2022 DE 102022113210
(71) Anmelder: B. Braun Melsungen AG, 34212 Melsungen (DE)
(72) Erfinder: SCHMOLL, Horst, 34302 Guxhagen (DE)
(74) Vertreter: Winter, Brandl - Partnerschaft mbB

(57) **Zusammenfassung**

Die vorliegende Offenbarung betrifft ein System (1) zur Vergabe einer Berechtigungsfreigabe an einem medizinischen Gerät (2) oder an zumindest einem ersten medizinischen Gerät (2) eines Gerätekomplexes (5), um Einstellungen an dem einen medizinischen Gerät (2) oder an dem zumindest einen ersten medizinischen Gerät eines Gerätekomplexes (5) vorzunehmen, mit einer Authentifizierungs-Servereinheit (3), die dazu vorgesehen und ausgebildet ist, um eine Authentifizierung durchzuführen, dem einen medizinischen Gerät (2) oder dem zumindest einen ersten medizinischen Gerät (2), und einer Authentisierungsvorrichtung (4), wobei die Authentisierungsvorrichtung (4) und/oder das eine medizinische Gerät (2) oder das zumindest eine erste medizinische Gerät (2) dazu vorgesehen und ausgebildet ist, um eine Authentisierung zumindest eines Nutzers durchzuführen, wobei das eine medizinische Gerät (2) oder das zumindest eine erste medizinische Gerät (2) und/oder die Authentisierungsvorrichtung (4) dazu vorgesehen und ausgelegt ist, um mit der Authentifizierungs-Servereinheit (3) zur Durchführung einer Authentifizierung des einen medizinischen Geräts (2) oder des zumindest einen ersten medizinischen Geräts (2) zu kommunizieren, wobei bei einer erfolgreichen Authentifizierung des einen medizinischen Geräts (2) oder des zumindest einen ersten medizinischen Geräts (2) die Authentifizierungs-Servereinheit (3) dazu vorgesehen und ausgebildet ist, um das eine medizinische Gerät oder das zumindest eine erste medizinische Gerät (2) mit einem nutzerspezifischen Nutzungslevel basierend auf Benutzerdaten freizugeben. Ferner betrifft die vorliegende Offenbarung ein Verfahren zur Vergabe einer Berechtigungsfreigabe an einem medizinischen Gerät (2) oder an zumindest einem ersten medizinischen Gerät (2) eines Gerätekomplexes (5), um Einstellungen an dem einen medizinischen Gerät (2) oder an dem zumindest einen ersten medizinischen Gerät eines Gerätekomplexes (5) vorzunehmen.

## Beschreibung

### Technisches Gebiet

Die vorliegende Offenbarung betrifft ein System und ein Verfahren zur Vergabe einer Berechtigungsfreigabe an einem medizinischen Gerät oder an zumindest einem ersten medizinischen Gerät eines Gerätekomplexes, um Einstellungen an dem einen medizinischen Gerät oder an dem zumindest einen ersten medizinischen Gerät eines Gerätekomplexes vorzunehmen.

### Hintergrund der Erfindung

Als Authentifizierung bezeichnet man allgemein die Prüfung eines Identitätsnachweises auf seine Authentizität. Im Beispiel eines Betriebssystems eines Geräts, welches Zugang zu einem gesicherten Bereich, beispielsweise einer Einstellung des Geräts gewähren kann, behauptet der Benutzer zunächst seine Zugangsberechtigung, indem er einen zuvor festgelegten, anonymen Benutzernamen eingibt. Zusätzlich authentisiert er sich, indem er ein ebenfalls zuvor festgelegtes Passwort oder einen Zahlencode angibt. Das Betriebssystem identifiziert daraufhin den Benutzer anhand dieser Angaben und führt anschließend die Authentifizierung durch, also die Verifizierung der erbrachten Behauptung über die Authentizität. Erst wenn diese Verifizierung erfolgreich ist, werden dem Benutzer die festgelegten Zugangsberechtigungen im Rahmen der Autorisierung üblicherweise für die Dauer einer Sitzung zugewiesen.

Bei bekannten Benutzerauthentifizierungsverfahren zur Benutzung eines beliebigen Geräts kann somit theoretisch jede beliebige Person auf das betreffende Gerät zugreifen sofern sie im Besitz von Passwort/Zahlencode und Benutzernamen ist. Vor diesem Hintergrund wäre beispielsweise im Fall eines medizinischen Geräts die tatsächliche Identität des Nutzers jedoch nicht bekannt und könnte für eine spätere Therapiedokumentation demzufolge auch nicht protokolliert werden. Im Hinblick auf notwendige Zugangsbeschränkungen aktueller medizinischer Geräte genügt beispielsweise ein mehr-, insbesondere vierstelliger Zahlencode gemäß allgemein bekannter Benutzerauthentifizierungsverfahren jedoch nicht den aktuellen Sicherheitsanforderungen in der Medizintechnik. Jede beliebige Person, welche im Besitz des entsprechenden Zahlencodes/Passworts und ggf. des anonymen Benutzernamens ist, wäre in der Lage auf das medizinische Gerät zuzugreifen, was nicht sein darf. Vielmehr ist es von erheblicher Bedeutung, dass nur ganz bestimmte, dafür vorgesehene Personen beispielsweise Einstellungen an dem jeweiligen medizinischen Gerät vornehmen können. Hierfür ist eine solche Authentifizierung notwendig, um nur einem bestimmten oder ausgewählten Benutzer(n) die Erlaubnis zum Vornehmen verschiedener Einstellungen an dem medizinischen Gerät zu gewähren.

### Stand der Technik

Aus der EP 3 087 771 B1 sind Systeme, Vorrichtungen und Verfahren bekannt, die die Authentifizierung für die Bedienung/Benutzung von Vorrichtungen innerhalb von Analytüberwachungssystemen ermöglichen. Die Analytüberwachungssysteme können In-vivo-Systeme sein und können eine Sensorsteuervorrichtung mit einem Sensor und einer begleitenden Schaltung sowie eine Lesevorrichtung zum Kommunizieren mit der Sensorsteuervorrichtung umfassen. Die Analytüberwachungssysteme können mit einem vertrauenswürdigen Computersystem verbunden werden, das sich an einem entfernten Standort befindet.

Die EP 3 859 573 A1 offenbart ein Verfahren zum automatischen Entsperren und/oder Sperren eines computergestützten Medizinprodukts in einem System. Das System umfasst das computergestützte Medizinprodukt und ein mobiles Endgerät, wobei das mobile Endgerät eine erste drahtlose Kommunikationsvorrichtung zum drahtlosen Senden und Empfangen von Daten umfasst, wobei mittels des mobilen Endgeräts eine mobile Anwendungssoftware ausführbar ist. Das Medizinprodukt umfasst einen Gerätecomputer, eine zweite drahtlose Kommunikationsvorrichtung zum drahtlosen Senden und Empfangen von Daten umfasst, wobei auf dem Gerätecomputer eine Treibersoftware ausführbar ist, die mittels der zweiten Kommunikationsvorrichtung des Medizinprodukts eine drahtlose Kommunikationsverbindung zu der ersten Kommunikationsvorrichtung des mobilen Endgeräts aufbauen kann. Hierbei ist es vorgesehen, dass das Medizinprodukt automatisch von einem gesperrtem Zustand in einen entsperrten Zustand versetzt wird, wenn die erste Kommunikationsvorrichtung des mobilen Endgeräts eine drahtlose Verbindung mit der zweiten Kommunikationsvorrichtung des Medizinprodukts aufgebaut hat und die von der zweiten Kommunikationsvorrichtung des Medizinprodukts empfangene Signalstärke des Signals der ersten Kommunikationsvorrichtung des mobilen Endgeräts einen vorbestimmten ersten Grenzwert überschreitet und/oder das Medizinprodukt automatisch von einem entsperrtem Zustand in einen gesperrten Zustand versetzt wird, wenn die erste Kommunikationsvorrichtung des mobilen Endgeräts eine drahtlose Verbindung mit der zweiten Kommunikationsvorrichtung des Medizinprodukts aufgebaut hat und die von der Kommunikationsvorrichtung des Medizinprodukts empfangene Signalstärke des Signals der ersten Kommunikationsvorrichtung des mobilen Endgeräts einen vorbestimmten zweiten Grenzwert unterschreitet und/oder wenn die drahtlose Verbindung länger als ein vorbestimmtes erstes Zeitintervall unterbrochen ist.

In der US 2017 0 140 134 ist schließlich ein beispielhaftes medizinisches Gerät beschrieben. Das Gerät umfasst ein physiologisches Messgerät, eine Gerätemanagement-Engine, eine Benutzer-Caching-Engine und eine Login-Engine. Die Gerätemanagement-Engine ist so konfiguriert, dass sie von dem physiologischen Messgerät erfasste Daten empfängt. Die Benutzer-Caching-Engine ist so konfiguriert, dass sie mit Benutzern verknüpfte Cache-Datensätze in einem Benutzer-Cache speichert. Die Login-Engine ist so konfiguriert, dass sie eine Benutzerkennung empfängt, die einem bestimmten Benutzer fest zugeordnet ist, und bestimmt, ob die Benutzerkennung einem Cache-Datensatz zugeordnet ist, der in dem Benutzer-Cache gespeichert ist. Wenn bestimmt wird, dass die Benutzerkennung mit einem Cache-Datensatz verknüpft ist, der im Benutzer-Cache gespeichert ist, wird die Login-Engine so konfiguriert, dass sie den Benutzer anmeldet. Wenn nicht festgestellt wird, dass die Benutzerkennung einem nicht abgelaufenen Cache-Datensatz zugeordnet ist, der im Benutzer-Cache gespeichert ist, ist die Login-Engine so konfiguriert, dass sie den Benutzer zur Eingabe eines Berechtigungsnachweises auffordert.

### Kurzbeschreibung der Offenbarung

Der vorliegenden Offenbarung liegt nunmehr die Aufgabe zugrunde, ein System bereitzustellen, welches eine einfache, schnelle sowie benutzerabhängige, sichere Authentifizierung ermöglicht. Ferner ist es insbesondere ein Ziel der vorliegenden Offenbarung, Nachteile aus dem Stand der Technik zu beseitigen oder zumindest zu verbessern.

Dies Aufgabe wird gelöst durch ein System mit den Merkmalen des Anspruchs 1 und einem Verfahren mit den Merkmalen des Anspruchs 9.

Demnach hat das System zur Vergabe einer Berechtigungsfreigabe an einem medizinischen Gerät oder an zumindest einem ersten medizinischen Gerät eines Gerätekomplexes, insbesondere einer Infusionspumpe, um Einstellungen an dem einen medizinischen Gerät oder an dem zumindest einen ersten medizinischen Gerät eines Gerätekomplexes vorzunehmen, eine Authentifizierungs-Servereinheit, die dazu vorgesehen und ausgebildet ist, um eine Authentifizierung durchzuführen, das eine medizinische Gerät oder das zumindest eine erste medizinische Gerät, und eine Authentisierungsvorrichtung, vorzugsweise in Form eines/ ein mobilen Endgerät. Hierbei ist die Authentisierungsvorrichtung und/oder das eine medizinische Gerät oder das zumindest eine erste medizinische Gerät dazu vorgesehen und ausgebildet, um eine Authentisierung zumindest eines Nutzers durchzuführen, wobei das eine medizinische Gerät oder das zumindest eine erste medizinische Gerät und/oder die Authentisierungsvorrichtung dazu vorgesehen und ausgelegt ist, um mit der Authentifizierungs-Servereinheit zur Durchführung einer Authentifizierung des einen medizinischen Geräts oder des zumindest einen ersten medizinischen Geräts zu kommunizieren. Bei einer erfolgreichen Authentifizierung des einen medizinischen Geräts oder des zumindest einen ersten medizinischen Geräts ist die Authentifizierungs-Servereinheit dazu vorgesehen und ausgebildet, um das eine medizinische Gerät oder das zumindest eine erste medizinische Gerät mit einem nutzerspezifischen Nutzungslevel basierend auf Benutzerdaten freizugeben.

In anderen Worten gewährt das System eine Einstellung von/an dem einem medizinischen Gerät bzw. von/ an dem zumindest einen ersten medizinischen Gerät, insbesondere einer Infusionspumpe. Hierbei ist eine Authentisierungsvorrichtung vorgesehen, in welche ein Nutzer seine Logindaten (Einloggdaten) bzw. Benutzername und Passwort eingibt. Alternativ oder zusätzlich kann die Eingabe der Logindaten bzw. des Benutzernamens und Passworts auch direkt an dem medizinischen Gerät, sofern es sich um ein einzelnes Gerät handelt, oder direkt an einem ersten medizinischen Gerät erfolgen, sofern es sich um einen Gerätekomplex handelt. In einem solchen Gerätekomplex ist hierbei ein erstes medizinisches Gerät vorgesehen, welches das sogenannte Hauptgerät ist, also das medizinische Gerät, mit dessen Hilfe die Authentisierung und Authentifizierung durchgeführt wird bzw. mit welchem eine erste Kommunikation oder eine erste Eingabe erfolgt. Nach einer Erfolgreichen Authentisierung, werden die Logindaten bzw. der Benutzername und das Passwort an die Authentifizierungs-Servereinheit weitergegeben/ gesendet, um die Authentifizierung durchzuführen, also zu überprüfen, ob Benutzername und Passwort korrekt sind und eine Vergabe einer Berechtigungsfreigabe zu erlauben ist. Sofern die Authentifizierung erfolgreich ist, wird eine Berechtigungsfreigabe zusammen mit einem entsprechenden nutzerspezifischen und vorzugsweise in der Authentifizierungs-Servereinheit hinterlegten Nutzungslevel an den Nutzer vergeben.

In anderen Worten ist ein System zur Benutzerauthentifizierung für ein medizinisches Gerät oder eine Gruppe medizinischer Geräte vorgesehen. Die anfängliche Authentisierung kann über eine Authentisierungsvorrichtung/ eine mobile Anwendung oder (direkt) auf dem einen medizinischen Gerät oder dem einen ersten medizinischen Gerät erfolgen. Nach einer erfolgreichen Authentifizierung mit Hilfe der Authentifizierungs-Servereinheit, gewährt die einem Benutzer zugeordnete Benutzungsstufe dem Nutzer entsprechend Zugriff auf eine Benutzeroberfläche des medizinischen Geräts bzw. des zumindest einen ersten medizinischen Geräts. Eine Authentisierungsvorrichtung/ eine mobile Anwendung kommuniziert mit der Authentifizierungs-Servereinheit/ mit einem IT-Server. Das eine medizinische Gerät bzw. das zumindest eine erste medizinische Gerät ist ebenfalls mit der Authentifizierungs-Servereinheit verbunden und es wird eine bidirektionale Kommunikation hergestellt. Der Benutzer gibt die Benutzerdaten in die Authentifizierungsvorrichtung ein. Alternativ ist es auch möglich, dass die Benutzerdaten direkt in das erste medizinische Gerät eingegeben werden.

In beiden Fällen ist es vorteilhaft, wenn Nutzername und Passwort oder biometrische Daten zur Benutzerauthentifizierung verwendet werden. Es ist bevorzugt, wenn die Authentisierungsvorrichtung dazu vorgesehen und ausgebildet ist, dass die Daten zur Benutzerauthentifizierung darin eingebbar sind. Diese Benutzerdaten/ Logindaten werden entweder von der Authentisierungsvorrichtung oder von dem einen/ersten medizinischen Gerät an die Authentifizierungs-Servereinheit gesendet.

Es ist bevorzugt, wenn die Authentifizierungs-Servereinheit dazu vorgesehen und ausgebildet ist, um zumindest ein weiteres mit dem zumindest einen ersten medizinischen Gerät in kommunikationskontakt stehendes medizinisches Gerät aus dem Gerätekomplex, vorzugsweise mit dem nutzerspezifischen Nutzungslevel, freizuschalten.

In anderen Worten sendet die Authentifizierungs-Servereinheit einen Authentifizierungsbefehl an zumindest ein weiteres medizinisches Gerät bzw. an alle mit dem ersten medizinischen Gerät in kommunikationskontakt stehenden medizinischen Geräten. Das bedeutet, es ist bevorzugt, wenn die weiteren medizinischen Geräte oder die Gruppe an medizinischen Geräten zu dem ersten medizinischen Gerät gehört. Hierbei ist eine Gruppe von 1 bis n medizinischen Geräten, die vom Server identifizierbar sind und zum Beispiel zum selben Bettenplatz oder zum selben Patienten gehören, bzw. in einem (Infusionspumpen-) Rack angeordnet sind.

Es ist von Vorteil, wenn das eine medizinische Gerät oder das zumindest eine erste medizinische Gerät oder die Authentisierungsvorrichtung dazu vorgesehen und ausgebildet ist, um die Benutzerdaten darin einzugeben und an die Authentifizierung-Servereinheit zu senden.

Es ist bevorzugt, wenn es sich bei dem mobilen Endgerät um ein Mobiltelefon oder ein Smartphone oder ein Tablet handelt.

Bevorzugt kann das mobile Endgerät mit biometrischen Authentifizierungselementen/ -sensoren, beispielsweise einem Fingerabdruckscanner, einem Irisscanner, einer Gesichtserkennung oder dergleichen ausgebildet sein.

Vorzugsweise beinhaltet das mobile Endgerät ein NFC-Lesegerät, welches personenbezogene Daten von einem Datenträger, beispielsweise einem Mitarbeiterausweis, auslesen kann und eine Identifizierung/ Authentifizierung des Bedieners ermöglicht. Insbesondere bevorzugt sendet das mobile Endgerät die personenbezogenen Daten an die Authentifizierungs-Servereinheit.

Es ist bevorzugt, wenn die Authentifizierungs-Servereinheit dazu vorgesehen und ausgebildet ist, die eingegebenen Daten zu überprüfen und zu validieren. Nach erfolgreicher Authentifizierung wird der Benutzer mit seinem Benutzerprofil verknüpft, welches Nutzungsebenen/ das Nutzungslevel für das eine medizinische Gerät oder das zumindest eine erste medizinische Gerät enthält/ aufweist.

Es ist von Vorteil, wenn die Nutzungsebenen/ das Nutzungslevel von dem in dem medizinischen Gerät vorgesehenen Medikament abhängt. In anderen Worten können die Nutzungsebenen variabel sein und an das Medikament angepasst sein.

Es ist bevorzugt, wenn es drei Nutzungsebenen gibt. Die erste Nutzungsebene kann ein Starten und ein Stoppen einer Medikamentenapplikation sowie ein Stummschalten von Alarmen ermöglichen. Die zweite Nutzungsebene kann zusätzlich ein Quittieren des Alarms ermöglichen. Die dritte Nutzungsebene kann ergänzend einen Medikamentenwechsel und/ oder eine Dosierungsänderung und/ oder eine Freigabe von Schmerzmedikamenten ermöglichen. Selbstverständlich können auch mehr oder weniger als drei Nutzungsebenen ausgebildet sein.

Weiterhin bevorzugt ist, wenn es eine Nutzungsebene gibt, welche eine Justierung einer patientengesteuerten Medikamentenapplikation ermöglicht.

Es ist vorteilhaft, wenn die Authentifizierungs-Servereinheit Zugriffe und/ oder durch den Benutzer vorgenommene Eingaben/ Änderungen protokoliert.

Es ist vorteilhaft, wenn die Authentifizierungs-Servereinheit dazu vorgesehen und ausgebildet ist, einen Authentifizierungsbefehl an das zumindest eine weitere medizinische Gerät zu senden, wobei der Authentifizierungsbefehl Informationen, vorzugsweise zumindest einen Benutzernamen, einen Zeitstempel und das nutzerspezifische Nutzungslevel, aufweist. In anderen Worten enthält der Authentifizierungsbefehl mindestens einen Benutzernamen, eine Benutzerstufe/ ein Nutzungslevel und einen Zeitstempel.

Es ist bevorzugt, wenn das eine medizinische Gerät oder das zumindest eine erste medizinische Gerät dazu vorgesehen und ausgebildet ist, um einen Code, vorzugsweise einen QR-Code oder Barcode, anzuzeigen, welcher dazu vorgesehen und ausgebildet ist, um durch Scannen mittels der Authentisierungsvorrichtung eine Geräteidentifikation des zumindest einen ersten medizinischen Geräts auszugeben.

Es ist von Vorteil, wenn eine Kommunikationsverbindung zwischen der Authentifizierungs-Servereinheit und dem einen medizinischen Gerät oder dem zumindest einen ersten medizinischen Gerät bidirektional ausgebildet ist.

Es ist vorteilhaft, wenn das zumindest eine weitere medizinische Gerät (2) dazu vorgesehen und ausgebildet ist, um die Informationen des Authentifizierungsbefehls, vorzugsweise in einem Prüfprotokoll des zumindest einen weiteren medizinischen Geräts, zu speichern. In anderen Worten, nach dem Empfang des Befehls/ Authentifizierungsbefehls ist das zumindest eine weitere medizinische Gerät dazu vorgesehen und ausgebildet, diesen Befehl im Prüfprotokoll des zumindest einen weiteren medizinischen Geräts zu speichern und um den Zugriff auf eine Benutzerschnittstelle entsprechend dem empfangenen Nutzungslevels/ der Benutzerstufe zu gewähren.

In anderen Worten ist es von Vorteil, dass, wenn sich der Benutzer an einem einzelnen oder ersten Gerät schon mal angemeldet hat, die Identifikation des einen medizinischen Geräts der Authentifizierungs-Servereinheit bereits bekannt ist. Im Falle der mobilen Anwendung/ der Authentisierungsvorrichtung gibt der Benutzer die Identifikationsnummer des medizinischen Geräts ein, beispielsweise durch eine manuelle Eingabe einer Nummer oder durch Scannen eines Barcodes, der die Nummer enthält. Es ist möglich Technologien wie RFID, Bluetooth oder NFC für die Geräteidentifizierung zu verwenden.

Es ist bevorzugt, wenn das System dazu vorgesehen und ausgebildet ist, um in einer Notfallsituation eine Bearbeitung des einen medizinischen Geräts oder des zumindest ersten und/ oder des zumindest einen weiteren medizinischen Geräts ohne Authentifizierung zu ermöglichen, vorzugsweise auf einem niedrigsten Nutzungslevel.

In anderen Worten muss das medizinische Gerät in Notfallsituationen den Zugriff auf der niedrigsten Benutzungsstufe ohne Authentifizierung erlauben. In diesem Fall wird ein anonymer Benutzername im Prüfprotokoll gespeichert, und dem Benutzer steht nur eine obligatorische Teilmenge der Gerätefunktionen zur Verfügung, die mit der niedrigsten Benutzungsstufe verbunden ist.

Es ist von Vorteil, wenn die Authentifizierungsvorrichtung dazu ausgebildet und vorgesehen ist, um zumindest Teile der Funktionalität des zumindest einen medizinischen Geräts bzw. der Gruppe medizinischer Geräte zu steuern.

Es ist vorteilhaft, wenn das zumindest eine medizinische Geräte dazu vorgesehen ist, um nach einer bestimmten Zeit der Inaktivität eine erneute Authentifizierung zu erfordern.

Es ist vorteilhaft, wenn die Authentifizierung eine zwei Faktor Authentifizierung ist. Vorzugsweise kann die zwei Faktor Authentifizierung mit einem Tan-Generator ausgebildet sein oder zwei beliebige unabhängige Authentifizierungsmethoden nutzen.

Darüber hinaus betrifft die vorliegende Offenbarung ein Verfahren zur Vergabe einer Berechtigungsfreigabe an einem medizinischen Gerät oder an zumindest einem ersten medizinischen Gerät eines Gerätekomplexes, insbesondere einer Infusionspumpe, um Einstellungen an dem einen medizinischen Gerät oder an dem zumindest einen ersten medizinischen Gerät eines Gerätekomplexes vorzunehmen, mit den folgenden Schritten:
- Eingabe von Benutzerdaten, vorzugsweise Benutzername und Passwort oder biometrischen Daten, in eine Authentisierungsvorrichtung und/oder das eine medizinische Gerät oder in das zumindest eine erste medizinische Gerät;
- Senden der Eingabe an eine Authentifizierungs-Servereinheit;
- Eingabe einer Geräteidentifikation des einen medizinischen Geräts oder des zumindest ersten medizinischen Geräts in die Authentisierungsvorrichtung, vorzugsweise durch Scannen eines Codes, welcher an dem einen medizinischen Gerät oder an dem zumindest ersten medizinischen Gerät anzeigbar ist;
- Durchführung der Authentifizierung an dem einen medizinischen Gerät oder an dem zumindest einen ersten medizinischen Gerät; und
- Vergabe der Berechtigungsfreigabe und Zuweisen eines nutzerspezifischen Nutzungslevels.

Es ist bevorzugt, wenn das Verfahren folgende Schritte aufweist:
- Senden eines Authentifizierungsbefehls an zumindest ein weiteres medizinisches Gerät;
- Speichern der empfangenen Informationen des Authentifizierungsbefehls in dem weiteren medizinischen Gerät; und
- Verwenden des zumindest einen weiteren medizinischen Geräts gemäß dem empfangenen Nutzungslevel.

In anderen Worten gibt ein Benutzer dessen Benutzerdaten in die Authentisierungsvorrichtung/ die mobile Anwendung oder in ein einzelnes (erstes) medizinisches Gerät ein. Die Benutzerdaten werden von der Authentisierungsvorrichtung oder dem einzelnen (ersten) medizinischen Gerät an die Authentifizierungs-Servereinheit gesendet und gewährt dem Nutzer den Zugang zu dem entsprechenden medizinischen Gerät.

Dem authentifizierten Benutzer wird eine Nutzungsstufe/ Nutzungslevel für das entsprechende medizinische Gerät zugewiesen. Anschließend wird in der mobilen Anwendung die Geräteidentifikation eingegeben. Die Eingabe erfolgt vorzugsweise per manueller Eingabe oder per Scannen eines Barcodes oder per Bluetooth.

Die Authentifizierungs-Servereinheit sendet einen Authentifizierungsbefehl an alle weiteren, mit dem ersten medizinischen Gerät in kommunikationskontakt stehenden bzw. der Gerätegruppe zugehörigen medizinischen Geräte. Der Authentifizierungsbefehl enthält den Benutzernamen, das Nutzungslevel und den Zeitstempel.

Die medizinischen Geräte speichern den empfangenen Benutzernamen und das Nutzungslevel in deren Prüfprotokoll. Die medizinischen Geräte erlauben die Benutzung entsprechend dem empfangenen Nutzerlevel.

Nach einer bestimmten Zeit der Inaktivität wird die Nutzung des Geräts wieder gesperrt, die Information wird in dem Prüfprotokoll gespeichert.

### Kurzbeschreibung der Figuren

Fig. 1 ist eine Darstellung zur Veranschaulichung eines Systems am Beispiel einer Gruppe von medizinischen Geräten bzw. Infusionspumpen gemäß einer Ausführungsform der vorliegenden Offenbarung; und
Fig. 2 ist ein Ablaufdiagramm des Verfahrens gemäß der vorliegenden Offenbarung.

Nachstehend werden Ausführungsbeispiele der vorliegenden Offenbarung auf der Basis der zugehörigen Figuren beschrieben.

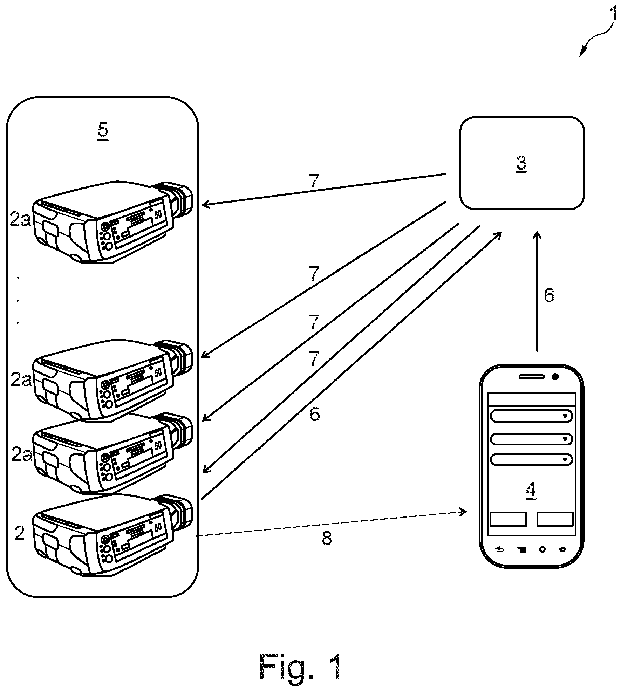

Fig. 1 ist eine Darstellung zur Veranschaulichung eines Systems 1 gemäß einer Ausführungsform der vorliegenden Offenbarung. Fig. 1 zeigt ein erstes medizinisches Gerät 2, eine Authentifizierungs-Servereinheit 3, und eine Authentisierungsvorrichtung 4. Ferner zeigt Fig. 1 weitere medizinische Geräte 2a, welche mit dem ersten medizinischen Gerät 2 in kommunikationskontakt stehen.

Das erste medizinische Gerät 2 sowie die weiteren damit in kommunikationskontakt stehenden medizinischen Geräte 2a sind vorzugsweise in einem Gerätekomplex/ Rack 5 angeordnet oder zumindest in einem Raum und zu einem Patienten zugehörig.

Das erste medizinische Gerät 2 und/oder die Authentisierungsvorrichtung 4 ist/sind dazu vorgesehen und ausgelegt, um mit der Authentifizierungs-Servereinheit 3 zur Durchführung einer Authentifizierung des ersten medizinischen Geräts 2 zu kommunizieren.

Bei einer erfolgreichen Authentifizierung des ersten medizinischen Geräts 2 ist die Authentifizierungs-Servereinheit 3 dazu vorgesehen und ausgebildet, um das erste medizinische Gerät 2 mit einem nutzerspezifischen Nutzungslevel basierend auf eingegebenen Benutzerdaten freizuschalten.

Zwischen dem ersten medizinischen Gerät 2 und der Authentifizierungs-Servereinheit 3 ist eine erste Kommunikationsverbindung 6 vorgesehen. Zusätzlich oder alternativ ist zwischen der Authentisierungsvorrichtung 4 und der Authentifizierungs-Servereinheit 3 eine erste Kommunikationsverbindung 6 vorgesehen. Die Authentifizierungs-Servereinheit 3 hat jeweils eine zweite Kommunikationsverbindung 7 mit dem ersten medizinischen Gerät 2 und den weiteren medizinischen Geräten 2a.

Die erste Kommunikationsverbindung 6 ist dazu ausgebildet und vorgesehen, um von einem Benutzer eingegebene Eingabedaten an die Authentifizierungs-Servereinheit 3 zu senden. Die zweite Kommunikationsverbindung 7 ist dazu ausgebildet und vorgesehen, um einen Authentifizierungsbefehl an das erste medizinische Gerät 2 und die weiteren medizinischen Geräte 2a zu senden. Die Kommunikationsverbindungen 6 und 7, welche das erste medizinische Gerät 2 und die Authentifizierungs-Servereinheit 3 miteinander verbinden, ist demnach eine bidirektionale Kommunikationsverbindung.

Wie in Fig. 1 gezeigt, ist die Authentisierungsvorrichtung 4 dazu vorgesehen und ausgebildet, um eine Geräteidentifikation 8 durchzuführen. Die Geräteidentifikation 8 erfolgt über eine manuelle Eingabe oder ein Scannen eines Barcodes oder per Bluetooth.

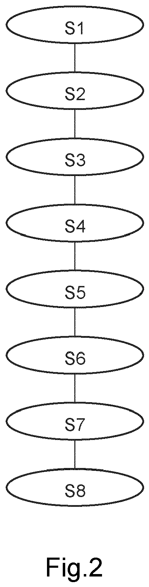

Fig. 2 ist ein Ablaufdiagramm des Verfahrens gemäß der vorliegenden Offenbarung.

In einem ersten Schritt S1 erfolgt die Eingabe von Benutzerdaten, vorzugsweise Benutzername und Passwort oder biometrischen Daten, in die Authentisierungsvorrichtung 4 oder in zumindest ein erstes medizinisches Gerät 2.

In einem zweiten Schritt S2 wird die Eingabe an eine Authentifizierungs-Servereinheit 3 gesendet. Die erhaltene Eingabe wird von der Authentifizierungs-Servereinheit 3 überprüft und validiert.

In einem dritten Schritt S3 wird eine Geräteidentifikation 8 des ersten medizinischen Geräts 2 durchgeführt. Dies erfolgt durch die Eingabe der Geräteidentifikation 8 in die Authentisierungsvorrichtung 4, vorzugsweise durch Scannen eines Codes, welcher an dem ersten medizinischen Gerät 2 anzeigbar ist.

In einem darauffolgenden Schritt S4 wird das erste medizinische Gerät 2 authentifiziert und in einem Schritt S5 wird ein benutzerspezifisches Nutzungslevel dem ersten medizinischen Gerät 2 zugwiesen.

In einem Schritt S6 sendet die Authentifizierungs-Servereinheit 3 einen Authentifizierungsbefehl an zumindest ein weiteres medizinisches Gerät 2a.

In Schritt S7 werden die empfangenen Informationen des Authentifizierungsbefehls in einem Prüfprotokoll eines jeden medizinischen Geräts 2 und 2a gespeichert. Die Informationen sind zumindest ein Benutzername, das Nutzungslevel und ein Zeitstempel.

In einem letzten Schritt S8 verwendet der Benutzer das zumindest eine medizinische Gerät 2 und/oder 2a gemäß dem empfangenen Nutzungslevel, um Einstellung vorzunehmen. Unter Verwenden des zumindest einen medizinischen Geräts 2 und/oder 2a wird das Einstellen vorhandener und/ oder benötigter Parameter verstanden.

### Bezugszeichenliste

- 1: Authentifizierungssystem
- 2: (erstes) medizinisches Gerät
- 2a: weitere medizinische Geräte
- 3: Authentifizierungs-Servereinheit
- 4: Authentisierungsvorrichtung
- 5: Gerätekomplex/Rack
- 6: Erste Kommunikationsverbindung
- 7: Zweite Kommunikationsverbindung
- 8: Geräteidentifikation

## Patentansprüche

1. System (1) zur Vergabe einer Berechtigungsfreigabe an einem medizinischen Gerät (2) oder an zumindest einem ersten medizinischen Gerät (2) eines Gerätekomplexes (5), insbesondere einer Infusionspumpe, um Einstellungen an dem einen medizinischen Gerät (2) oder an dem zumindest einen ersten medizinischen Gerät eines Gerätekomplexes (5) vorzunehmen, mit:
- einer Authentifizierungs-Servereinheit (3), die dazu vorgesehen und ausgebildet ist, um eine Authentifizierung durchzuführen,
- dem einen medizinischen Gerät (2) oder dem zumindest einen ersten medizinischen Gerät (2), und
- einer Authentisierungsvorrichtung (4), vorzugsweise einem mobilen Endgerät, wobei die Authentisierungsvorrichtung (4) und/oder das eine medizinische Gerät (2) oder das zumindest eine erste medizinische Gerät (2) dazu vorgesehen und ausgebildet ist, um eine Authentisierung zumindest eines Nutzers durchzuführen, wobei
das eine medizinische Gerät (2) oder das zumindest eine erste medizinische Gerät (2) und/oder die Authentisierungsvorrichtung (4) dazu vorgesehen und ausgelegt ist, um mit der Authentifizierungs-Servereinheit (3) zur Durchführung einer Authentifizierung des einen medizinischen Geräts (2) oder des zumindest einen ersten medizinischen Geräts (2) zu kommunizieren,
**dadurch gekennzeichnet, dass**
bei einer erfolgreichen Authentifizierung des einen medizinischen Geräts (2) oder des zumindest einen ersten medizinischen Geräts (2) die Authentifizierungs-Servereinheit (3) dazu vorgesehen und ausgebildet ist, um das eine medizinische Gerät oder das zumindest eine erste medizinische Gerät (2) mit einem nutzerspezifischen Nutzungslevel basierend auf Benutzerdaten freizugeben.

2. System (1) gemäß Anspruch 1, **dadurch gekennzeichnet, dass** die Authentifizierungs-Servereinheit (3) dazu vorgesehen und ausgebildet ist, um zumindest ein weiteres mit dem zumindest einen ersten medizinischen Gerät (2) in kommunikationskontakt stehendes medizinisches Gerät (2a) aus dem Gerätekomplex (5), vorzugsweise mit dem nutzerspezifischen Nutzungslevel, freizuschalten.

3. System (1) gemäß Anspruch 1 oder 2, **dadurch gekennzeichnet, dass** das eine medizinische Gerät (2) oder das zumindest eine erste medizinische Gerät (2) oder die Authentisierungsvorrichtung (4) dazu vorgesehen und ausgebildet ist, um die Benutzerdaten darin einzugeben und an die Authentifizierung-Servereinheit (3) zu senden.

4. System (1) gemäß Anspruch 2 oder 3, **dadurch gekennzeichnet, dass** die Authentifizierungs-Servereinheit (3) dazu vorgesehen und ausgebildet ist, einen Authentifizierungsbefehl an das zumindest eine weitere medizinische Gerät (2a) zu senden, wobei der Authentifizierungsbefehl Informationen, vorzugsweise zumindest einen Benutzernamen, einen Zeitstempel und das nutzerspezifische Nutzungslevel, aufweist.

5. System (1) gemäß einem der Ansprüche 1 bis 4, **dadurch gekennzeichnet, dass** das eine medizinische Gerät (2) oder das zumindest eine erste medizinische Gerät dazu vorgesehen und ausgebildet ist, um einen Code, vorzugsweise einen QR-Code oder Barcode, anzuzeigen, welcher dazu vorgesehen und ausgebildet ist, um durch Scannen mittels der Authentisierungsvorrichtung eine Geräteidentifikation (8) des zumindest einen ersten medizinischen Geräts (2a) auszugeben.

6. System (1) gemäß einem der Ansprüche 1 bis 5, **dadurch gekennzeichnet, dass** eine Kommunikationsverbindung (6, 7) zwischen der Authentifizierungs-Servereinheit (3) und dem einen medizinischen Gerät (2) oder dem zumindest einen ersten medizinischen Gerät (2) bidirektional ausgebildet ist.

7. System (1) gemäß einem der Ansprüche 4 bis 6, **dadurch gekennzeichnet, dass** das zumindest eine weitere medizinische Gerät (2a) dazu vorgesehen und ausgebildet ist, um die Informationen des Authentifizierungsbefehls, vorzugsweise in einem Prüfprotokoll des zumindest einen weiteren medizinischen Geräts (2a), zu speichern.

8. System (1) gemäß einem der Ansprüche 2 bis 7, **dadurch gekennzeichnet, dass** das System (1) dazu vorgesehen und ausgebildet ist, um in einer Notfallsituation eine Bearbeitung des einen medizinischen Geräts (2) oder des zumindest ersten und/ oder des zumindest einen weiteren medizinischen Geräts (2, 2a) ohne Authentifizierung zu ermöglichen, vorzugsweise auf dem niedrigsten Nutzungslevel.

9. Verfahren zur Vergabe einer Berechtigungsfreigabe an einem medizinischen Gerät (2) oder an zumindest einem ersten medizinischen Gerät (2) eines Gerätekomplexes (5), insbesondere einer Infusionspumpe, um Einstellungen an dem einen medizinischen Gerät (2) oder an dem zumindest einen ersten medizinischen Gerät eines Gerätekomplexes (5) vorzunehmen, mit den folgenden Schritten:
- Eingabe von Benutzerdaten, vorzugsweise Benutzername und Passwort oder biometrischen Daten, in eine Authentisierungsvorrichtung (4) und/oder das eine medizinische Gerät (2) oder in das zumindest eine erste medizinische Gerät (2);
- Senden der Eingabe an eine Authentifizierungs-Servereinheit (3);
- Eingabe einer Geräteidentifikation des einen medizinischen Geräts (2) oder des zumindest ersten medizinischen Geräts (2) in die Authentisierungsvorrichtung (4), vorzugsweise durch Scannen eines Codes, welcher an dem einen medizinischen Gerät (2) oder an dem zumindest ersten medizinischen Gerät (2) anzeigbar ist;
- Durchführung der Authentifizierung an dem einen medizinischen Gerät (2) oder an dem zumindest einen ersten medizinischen Gerät (2); und
- Vergabe der Berechtigungsfreigabe und Zuweisen eines nutzerspezifischen Nutzungslevels.

10. Authentifizierungsverfahren gemäß Anspruch 9, **gekennzeichnet, durch** die folgenden weiteren Schritte:
- Senden eines Authentifizierungsbefehls an zumindest ein weiteres medizinisches Gerät (2a);
- Speichern der empfangenen Informationen des Authentifizierungsbefehls in dem weiteren medizinischen Gerät (2a); und
- Verwenden des zumindest einen weiteren medizinischen Geräts (2a) gemäß dem empfangenen Nutzungslevel.
